# EUROPEAN PATENT APPLICATION

(11) **EP 3 524 624 A1**
(43) Date of publication of application: **14.08.2019**
(21) Application number: 17854915.0
(22) Date of filing: 28.09.2017
(51) Int. Cl.: C07K 16/28, A61K 39/395, A61P 35/00, A61P 37/00, C12N 1/15, C12N 1/21, C12N 5/10, C12N 15/09, C12P 21/08

(54) **ANTI-CD27 ANTIBODY, ANTIGEN-BINDING FRAGMENT THEREOF, AND MEDICAL USE OF SAME**

(30) Priority: 29.09.2016 CN 201610865648
(71) Applicant: Jiangsu Hengrui Medicine Co., Ltd., Jiangsu 222047 (CN)
(72) Inventor: YAN, Shude, Lianyungang Jiangsu222047 (CN); JIANG, Jiahua, Lianyungang Jiangsu 222047 (CN); ZHANG, Lianshan, Lianyungang Jiangsu 222047 (CN); CAO, Guoqing, Lianyungang Jiangsu 222047 (CN)
(74) Representative: Gevers & Orès
(86) International application number: PCT/CN2017/103842
(87) International publication number: WO 2018/059465

(57) **Abstract**

An anti-CD27 antibody, an antigen-binding fragment thereof, and a medical use of the same. Further, provided are a chimeric antibody comprising a CDR of the anti-CD27 antibody, a humanized antibody, a pharmaceutical composition comprising a human anti-CD27 antibody or an antigen-binding fragment thereof, and a use of the same as an anti-cancer drug. The present invention particularly relates to a human anti-CD27 antibody and a use thereof in preparing a drug for treating a CD27-mediated disease or disorder.

## Description

### FIELD OF THE INVENTION

The present invention relates to a CD27 antibody, antigen-binding fragment thereof, a chimeric antibody or a humanized antibody comprising the CDR regions of the CD27 antibody, as well as pharmaceutical composition comprising the human CD27 antibody and the antigen-binding fragment thereof, as well as its use as an anti-cancer drug.

### BACKGROUND OF THE INVENTION

Tumor immunotherapy has been a hot area in the filed of anti-cancer drugs for a long time. Tumor immunotherapy kills and destroys tumors by fully utilizing and mobilizing killer T cells in tumor patients. It may be one of the most effective and safest ways for cancer treatment. A human T cell is activated *in vivo* via a two-signaling-pathway system, wherein antigen-presenting cells are needed to present MHC-antigen peptide to T cells to provide a first signal. Then a series of co-stimulatory molecules are required to provide a second signal to enable T cells to exhibit a normal immune response.

The interaction between T cells and antigen-presenting cells requires a variety of accessory molecules that facilitate the generation of an immune response, and CD27 is an important one of them. CD27 is a member of the tumor necrosis factor receptor (TNF-R) superfamily and binds to CD70 on the surface of T cells. CD27 is a glycosylated type I transmembrane protein, usually a homodimer linked by a disulfide bridge which is located at the membrane-proximal region of extracellular domain (Camerini et.al., J Immunol. 147: 3165-69, 1991). The CD27 antigen cross-linked on T cells can provide co-stimulation signal, while T cell proliferation and cellular immune activation can be induced when CD27 is cross-linked with T cell receptors.

CD27 is expressed on mature thymocytes, most CD4+ and CD8+ peripheral blood T cells, natural killer cells and B cells, and is also highly expressed in B cell non-Hodgkin's lymphoma and B cell chronic lymphocytic leukemia; increased levels of soluble CD27 protein are also observed in serum or at the active sites of the diseases in infections such as parasites and cytomegalovirus (CMV), multiple sclerosis, sarcoidosis, and B-cell chronic lymphocytic leukemia (Kobata T et.al, Proc. Natl. Acad. Sci. USA. 1995 (24): 11249-53; Ranheim EA et.al, Blood. 1995 85 (12): 3556-65; Loenen WA et.al, Eur. J. Immunol. 1992 22: 447).

Recent studies have shown that anti-CD27 monoclonal antibody agonists can promote T cell responses and can be used as potential anti-cancer therapeutics (see patents WO2008051424, WO2011130434). Although the results obtained to date have identified CD27 as a useful target for immunotherapy, it remains unknown which specific features of anti-CD27 monoclonal antibody are particularly beneficial for treatment. There is still a need in the art to understand in depth which specific functional properties of anti-CD27 antibody are therapeutically effective, so as to further obtain potentially improved anti-CD27 antibodies that are more effective against diseases.

Currently, a number of international companies have developed tumor antibody drugs for the CD27 target. The anti-CD27 antibody from US Celldex has been in Phase II clinical trials, the related products of companies such as Merck, Aduro and Apogenix are still in phase of pre-clinical development.

The present invention intends to provide anti-CD27 antibodies with high affinity, high selectivity and high biological activity, with specific functional properties that can be associated with the desired beneficial therapeutic effects.

### SUMMARY OF THE INVENTION

The present invention provides an anti-CD27 antibody or antigen-binding fragment thereof comprising:
an antibody light chain variable region comprising at least one LCDR as shown in the sequences selected from SEQ ID NO: 6, SEQ ID NO: 7, SEQ ID NO: 8; SEQ ID NO : 14, SEQ ID NO: 15 or SEQ ID NO: 16; and
an antibody heavy chain variable region comprising at least one HCDR as shown in the sequences selected from SEQ ID NO: 3, SEQ ID NO: 4, SEQ ID NO: 5, SEQ ID NO :11, SEQ ID NO: 12 or SEQ ID NO: 13.

In a preferred embodiment of the present invention, the anti-CD27 antibody or antigen-binding fragment thereof as described above, wherein the antibody light chain variable region comprises LCDR1 as shown in SEQ ID NO: 6 or SEQ ID NO: 14.

In a preferred embodiment of the present invention, the anti-CD27 antibody or antigen-binding fragment thereof as described above, wherein the antibody light chain variable region comprises LCDR2 as shown in SEQ ID NO: 7 or SEQ ID NO: 15.

In a preferred embodiment of the present invention, the anti-CD27 antibody or antigen-binding fragment thereof as described above, wherein the antibody light chain variable region comprises LCDR3 as shown in SEQ ID NO: 8 or SEQ ID NO: 16.

In a preferred embodiment of the present invention, the anti-CD27 antibody or antigen-binding fragment thereof as described above, wherein the antibody heavy chain variable region comprises HCDR1 as shown in SEQ ID NO:3 or SEQ ID NO:11.

In a preferred embodiment of the present invention, the anti-CD27 antibody or antigen-binding fragment thereof as described above, wherein the antibody heavy chain variable region comprises HCDR2 as shown in SEQ ID NO:4 or SEQ ID NO:12.

In a preferred embodiment of the present invention, the anti-CD27 antibody or antigen-binding fragment thereof as described above, wherein the antibody heavy chain variable region comprises HCDR3 as shown in SEQ ID NO:5 or SEQ ID NO:13.

In a preferred embodiment of the present invention, the anti-CD27 antibody or antigen-binding fragment thereof as described above, wherein the antibody light chain variable region comprises LCDR1, LCDR2 and LCDR3 as shown in SEQ ID NO: 6, SEQ ID NO: 7 and SEQ ID NO: 8 respectively.

In a preferred embodiment of the present invention, the anti-CD27 antibody or antigen-binding fragment thereof as described above, wherein the antibody light chain variable region comprises LCDR1, LCDR2 and LCDR3 as shown in SEQ ID NO: 14, SEQ ID NO: 15 and SEQ ID NO: 16 respectively.

In a preferred embodiment of the present invention, the anti-CD27 antibody or antigen-binding fragment thereof as described above, wherein the antibody heavy chain variable region comprises HCDR1, HCDR2 and HCDR3 as shown in SEQ ID NO: 3, SEQ ID NO: 4 and SEQ ID NO: 5 respectively.

In a preferred embodiment of the present invention, the anti-CD27 antibody or antigen-binding fragment thereof as described above, wherein the antibody heavy chain variable region comprises HCDR1, HCDR2 and HCDR3 as shown in SEQ ID NO: 11, SEQ ID NO: 12 and SEQ ID NO: 13 respectively.

In a preferred embodiment of the present invention, the anti-CD27 antibody or antigen-binding fragment thereof as described above, wherein the antibody light chain variable region comprises:
LCDR1, LCDR2 and LCDR3 as shown in SEQ ID NO: 6, SEQ ID NO: 7 and SEQ ID NO: 8 respectively; or
LCDR1, LCDR2 and LCDR3 as shown in SEQ ID NO: 14, SEQ ID NO: 15 and SEQ ID NO: 16 respectively;
and wherein the antibody heavy chain variable region comprises:
   HCDR1, HCDR2 and HCDR3 as shown in SEQ ID NO: 3, SEQ ID NO: 4 and SEQ ID NO: 5 respectively; or
   HCDR1, HCDR2 and HCDR3 as shown in SEQ ID NO: 11, SEQ ID NO: 12 and SEQ ID NO: 13 respectively.

A particularly preferred anti-CD27 antibody or antigen-binding fragment thereof can be selected from any of the following:
(1) the antibody light chain variable region comprises LCDR1, LCDR2 and LCDR3 as shown in SEQ ID NO: 6, SEQ ID NO: 7 and SEQ ID NO: 8 respectively; the antibody heavy chain variable region comprises HCDR1, HCDR2 and HCDR3 as shown in SEQ ID NO: 3, SEQ ID NO: 4 and SEQ ID NO: 5 respectively.
(2) the antibody light chain variable region comprises LCDR1, LCDR2 and LCDR3 as shown in SEQ ID NO: 14, SEQ ID NO: 15 and SEQ ID NO: 16 respectively; the antibody heavy chain variable region comprises LCDR1, LCDR2 and LCDR3 as shown in SEQ ID NO: 11, SEQ ID NO: 12 and SEQ ID NO: 13 respectively.

In a preferred embodiment of the present invention, the sequence of the antibody light chain variable region is selected from SEQ ID NO: 2 or SEQ ID NO: 10; the sequence of heavy chain variable region is selected from SEQ ID NO: 1 or SEQ ID NO: 9.

A particularly preferred anti-CD27 antibody or antigen-binding fragment thereof can be selected from any of the following:
(1) the sequence of the antibody light chain variable region is SEQ ID NO: 2, the sequence of the heavy chain variable region is SEQ ID NO: 1.
(2) the sequence of the antibody light chain variable region is SEQ ID NO: 10, the sequence of the heavy chain variable region is SEQ ID NO: 9.

In a preferred embodiment of the present invention, the anti-CD27 antibody or antigen-binding fragment thereof as described above, wherein the antibody or antigen-binding fragment thereof is a murine antibody or fragment thereof.

In a preferred embodiment of the present invention, the anti-CD27 antibody or antigen-binding fragment thereof as described above, wherein the antibody or antigen-binding fragment thereof is a chimeric antibody or fragment thereof.

In a preferred embodiment of the present invention, the anti-CD27 antibody or antigen-binding fragment thereof as described above, wherein the antibody or antigen-binding fragment thereof is a humanized antibody or fragment thereof.

In a preferred embodiment of the present invention, the anti-CD27 antibody or antigen-binding fragment thereof as described above, wherein the antibody or antigen-binding fragment thereof is a human antibody or fragment thereof.

In a preferred embodiment of the present invention, the anti-CD27 antibody or antigen-binding fragment thereof as described above further comprises a constant region derived from human IgG1, IgG2, IgG3, or IgG4, or a variant thereof, preferably comprises a constant region derived from human IgG1, IgG2 or IgG4. IgG2 or IgG4 does not have ADCC toxicity, or IgG1 without ADCC (antibody-dependent cell-mediated cytotoxicity) can be used, due to amino acid mutation.

In a preferred embodiment of the present invention, the anti-CD27 antibody or antigen-binding fragment thereof as described above, wherein the antigen-binding fragment is Fab, Fv, sFv, F(ab')2.

The present invention further provides a DNA sequence encoding the anti-CD27 antibody or antigen-binding fragment thereof as described above.

The present invention further provides an expression vector comprising the DNA sequence as described above.

The present invention further provides a host cell transformed with the expression vector as described above.

In a preferred embodiment of the present invention, the host cell as described above, characterized in that the host cell is bacterium, preferably is *E. coli.*

In a preferred embodiment of the present invention, the host cell as described above is yeast, preferably is *pichia pastoris.*

In a preferred embodiment of the present invention, the host cell as described above is mammalian cell, preferably is Chinese hamster ovary (CHO) cell or human embryonic kidney (HEK) 293 cell.

The present invention further provides a pharmaceutical composition comprising the anti-CD27 antibody or antigen-binding fragment thereof as described above and a pharmaceutically acceptable excipient, dilution or carrier.

The present invention further provides a multispecific antibody comprising the light chain variable region and the heavy chain variable region as described above.

The present invention further provides a single chain antibody comprising the light chain variable region and the heavy chain variable region as described above.

The present invention further provides an antibody-drug conjugate comprising the light chain variable region and the heavy chain variable region as described above. The antibody-drug conjugates are well known in the art and are formed by the interconnection of antibody-linker-drug (toxin), known linkers include cleavable linkers, non-cleavable linkers, such as the linkers including, but not limited to, SMCC, SPDP etc.; toxins are also well known in the art, such as DM1, DM4, MMAE, MMAF and the like.

The present invention further provides use of the anti-CD27 antibody or antigen-binding fragment thereof as described above, or the pharmaceutical composition comprising the same, including but not limited to in form of multispecific antibody, single chain antibody, antibody-drug conjugate, in the preparation of a medicament for treating or preventing a CD27 mediated disease or condition; wherein, the disease is preferably cancer and autoimmune disease; more preferably is CD27-expressing cancer and autoimmune disease; the cancer is most preferably rectal cancer, brain cancer, kidney cancer, lung cancer, liver cancer, multiple myeloma and melanin; the autoimmune disease is most preferably autoimmune encephalomyelitis, systemic lupus erythematosus, multiple sclerosis and rheumatoid arthritis.

The present invention further provides a method of treating and preventing CD27 mediated disease or condition, the method comprising administering a therapeutically effective amount of the anti-CD27 antibody or antigen-binding fragment thereof as described above, or the pharmaceutical composition comprising the same, including but not limited to in form of multispecific antibody, single chain antibody, antibody-drug conjugate, to the patients in need thereof; wherein, the disease is preferably cancer and autoimmune disease; more preferably is CD27-expressing cancer and autoimmune disease; the cancer is most preferably rectal cancer, brain cancer, kidney cancer, lung cancer, liver cancer, multiple myeloma and melanin; the autoimmune disease is most preferably autoimmune encephalomyelitis, systemic lupus erythematosus, multiple sclerosis and rheumatoid arthritis.

### BRIEF DESCRIPTION OF THE DRAWINGS

Figure 1: Affinity and kinetic parameters of the binding of anti-CD27 antibodies mAb076 and mAb077 to the antigen, which were determined using the method described in the corresponding Examples by Data Acquisition 7.1 software.
Figure 2: Data showing the binding of human anti-CD27 antibody mAb076 and mAb077 to human or mouse CD27 antigen expressed by HEK293 cells, but not binding to the rest of TNFR superfamily receptors, determined by flow cytometry (The proportion of cells with binding signals, relative to the total number of cells).
Figure 3: Gradient dilution titration curves showing the binding of anti-CD27 antibody mAb076 to human or mouse CD27 antigen expressed by HEK293 cells, which were determined by flow cytometry.
Figure 4: Data showing the binding of human anti-CD27 antibody mAb076 and mAb077 to mouse T cells, which were determined by flow cytometry (The proportion of cells with binding signals, relative to the total cells).
Figure5: Data showing that antibodies mAb076 and mAb077 effectively block the binding of CD27 expressed on cells to its ligand CD70, which were determined by flow cytometry (The proportion of cells with CD70-specific binding signals, relative to the total cells).
Figure 6: Gradient dilution titration curves of activation of the NF-kB signaling pathway by antibodies mAb076 and mAb077, detected using the luciferase reporter gene method.
Figure 7: Image of human peripheral blood mononuclear T cells activated by antibodies mAb076 and mAb077 determined by flow cytometry: (A). Promotion effect of 1F5, mAb076 and mAb077 on IFN-γ expression of T cells. (B). Activation of T cell proliferation by mAb076 antibody.
Figure 8: Affinity and kinetic parameters of the binding of anti-CD27 antibodies mAb076 and mAb077 to FcγRI receptor, which were determined using the method described in the corresponding Examples by Data Acquisition 7.1 software.
Figure 9: Tumor size of E.G7-OVA transplanted T lymphoma in vehicle-treated mice, mice treated with control human IgG1 antibody or mice treated with anti-CD27 mAb076-IgG antibody, relative to days after tumor inoculation (in mm³). The tumor growth curve is represented in (A); the relative change in body weight of the mouse is represented in (B). The error is expressed as standard error.
Figure 10: Tumor size of human Raji transplanted lymphoma in PBS-treated mice, mice treated with control human IgG1 antibody or mice treated with anti-CD27 mAb076-IgG antibody, relative to days after tumor inoculation (in mm³). The tumor growth curve is represented in (A); the relative change in body weight of the mouse is represented in (B). The error is expressed as standard error.

### DETAILED DESCRIPTION OF THE INVENTION

### 1. TERMS

In order to more readily understand the present invention, certain technical and scientific terms are specifically defined below. Unless specifically indicated elsewhere in this document, all other technical and scientific terms used herein have the meaning commonly understood by one of ordinary skilled in the art to which this invention pertains.

As used herein, the single-letter code and the three-letter code for amino acids are as described in J. Biol. Chem, 243, (1968) p3558.

As used herein, "Antibody" refers to immunoglobulin, a structure of four-peptide chains connected together by disulfide bonds between two identical heavy chains and two identical light chains. Different immunoglobulin heavy chain constant regions exhibit different amino acid compositions and sequence orders, hence present different kinds of antigenicity. Accordingly, immunoglobulins can be divided into five categories, or called as immunoglobulin isotypes, namely IgM, IgD, IgG, IgA and IgE, their corresponding heavy chains are µ chain, δ chain, γ chain, α chain and ε chain, respectively. According to its amino acid composition of hinge region and the number and location of heavy chain disulfide bonds, the same type of Ig can be divided into different sub-categories, for example, IgG can be divided into IgG1, IgG2, IgG3, and IgG4. Light chains can be divided into κ or λ chain, due to different constant regions. Each IgG among the five types has κ or λ chain.

In the present invention, the antibody light chain variable region mentioned herein further comprises a light chain constant region, which comprises a human or murine κ, λ chain or a variant thereof.

In the present invention, the antibody heavy chain variable region mentioned herein further comprises a heavy chain constant region, which comprises human or murine IgG1, IgG2, IgG3, IgG4 or a variant thereof.

The sequence of about 110 amino acids close to the N-terminal of the antibody heavy and light chains, changes largely, known as variable region (V region); the rest sequence of amino acid close to the C-terminal is relatively stable, known as constant region (C region). Variable region comprises three hypervariable regions (HVR) and four framework regions (FR) having relatively conserved sequence. Three hypervariable regions determine the specificity of the antibody, also known as complementarity determining region (CDR). Each light chain variable region (VL) and each heavy chain variable region (VH) is composed of three CDR regions and four FR regions, with sequential order from the amino terminal to the carboxyl terminal being: FR1, CDR1, FR2, CDR2, FR3, CDR3, and FR4. Three light chain CDRs refer to LCDR1, LCDR2, and LCDR3; three heavy chain CDRs refer to HCDR1, HCDR2 and HCDR3. The number and location of CDR amino acid residues in VL and VH regions of the antibody or antigen binding fragment herein comply with known Kabat numbering criteria (LCDR1-3, HCDR2-3), or comply with Kabat and Chothia numbering criteria (HCDR1).

The term "antigen presenting cell" or "APC" is a cell which displays a foreign antigen complexed with MHC on its surface. T cells recognize this complex using the T cell receptor (TCR). Examples of APCs include, but are not limited to, dendritic cells (DC), peripheral blood mononuclear cells (PBMC), monocytes, B lymphoblasts and monocyte-derived dendritic cells (DC). The term "antigen presentation" refers to the process by which APCs capture antigens and enable them to be recognized by T cells, for example as a component of MHC-I/MHC-II conjugates.

The term "CD27" refers to a cell surface receptor that is a member of the TNF receptor superfamily. CD27 is a molecule essential for the production and long-term maintenance of T cell immunity and plays a key role in regulating B cell activation and immunoglobulin synthesis. The term "CD27" includes any variant or isoform of CD27 that is naturally expressed by a cell (for example, the hunman CD27 registered in GENBANK under the accession number AAH12160). The antibodies of the present invention can be cross-reactive with CD27 from non-human species. Alternatively, the antibody may also be specific for human CD27 and may not exhibit cross-reactivity with other species. CD27 or any variant or isoform thereof can be isolated from cells or tissues in which they are naturally expressed, or produced by recombinant techniques using common techniques in the art and those described herein. Preferably, the anti-CD27 antibody targets human CD27 with a normal glycosylation pattern.

The term "human antibody" includes antibodies having variable and constant regions of human germline immunoglobulin sequences. Human antibodies of the present invention can include amino acid residues that are not encoded by human germline immunoglobulin sequences (such as mutations introduced by random or site-specific mutagenesis *in vitro* or by somatic mutation *in vivo).* However, the term "human antibody" does not include an antibody in which a CDR sequence derived from a germline of another mammalian species, such as a mouse, has been grafted into a human framework sequence (i.e. "humanized antibody").

The term "humanized antibody", also known as CDR-grafted antibody, refers to an antibody generated by grafting murine CDR sequences into a variable region framework of a human antibody. Humanized antibody overcomes the strong antibody response induced by the chimeric antibody that carries a large amount of murine protein components. To avoid the decrease in activity caused by reducing the immunogenicity, the variable region of the human antibody is subjected to minimum back-mutation to maintain the activity.

The term "chimeric antibody", is an antibody which is formed by fusing the variable region of a murine antibody with the constant region of human antibody, so as to alleviate the murine antibody-induced immune response. To establish a chimeric antibody, a hybridoma secreting specific murine monoclonal antibody is first established, variable region genes are then cloned from murine hybridoma cells, and then constant region genes of human antibody are cloned as desired, the murine variable region genes are ligated with human constant region genes to form a chimeric gene which can be inserted into a human vector, and finally the chimeric antibody molecule is expressed in a eukaryotic or prokaryotic industrial system. The constant region of a human antibody is selected from the heavy chain constant region derived from human IgG1, IgG2, IgG3 or IgG4, or a variant thereof, and preferably comprises a heavy chain constant region derived from human IgG2 or IgG4, or as an alternative IgG1 without ADCC (antibody-dependent cell-mediated cytotoxicity) can be used, since IgG1 has been subjected to amino acid mutation.

As used herein, "antigen-binding fragment" refers to Fab fragment, Fab' fragment, F(ab')2 fragment with antigen-binding activity, as well as Fv fragment scFv fragment binding with human CD27; it comprises one or more CDR regions of antibodies described in the present invention, selected from the group consisting of SEQ ID NO:3 to SEQ ID NO:8. Fv fragment is a minimum antibody fragment comprising a heavy chain variable region, a light chain variable region, and all antigen-binding sites without a constant region. Generally, Fv antibody further comprises a polypeptide linker between the VH and VL domains and is capable of forming a structure required for antigen binding. Also, different linkers can be used to connect two variable regions of antibody to form a polypeptide, named single chain antibody or single chain Fv (sFv). As used herein, the term "binding with CD27" means capable of interacting with human CD27. As used herein, the term "antigen-binding sites" of the present invention refers to discontinuous, three-dimensional sites on the antigen, recognized by the antibody or the antigen-binding fragment of the present invention.

The term "multispecific antibody" is used in its broadest sense to encompass antibodies having multi-epitope specificity. These multispecific antibodies include, but are not limited to, antibodies comprising a heavy chain variable region (VH) and a light chain variable region (VL), wherein the VH-VL unit has multi-epitope specificity; antibodies having two or more VL and VH regions, each VH-VL unit binding to different target or different epitope of the same target; antibodies having two or more single variable regions, each single variable region binding to different target or different epitope of the same target; full length antibodies, antibody fragments, diabodies, bispecific diabodies and triabodies, antibody fragments that have been covalently or non-covalently linked, and the like.

The term "antibody-drug conjugate" (ADC) refers to one or more heterologous chemically synthesized molecules, including but not limited to the antibody or antibody fragment conjugated to cytotoxic agents.The term "single-chain antibody" is a single-chain recombinant protein linked by a linker peptide between the heavy chain variable region (VH) and the light chain variable region (VL) of an antibody. It is the smallest antibody fragment with complete antigen binding site.

The term "epitope" refers to a site on an antigen that is specifically bound by an immunoglobulin or antibody. Epitopes may be formed from adjacent amino acids or nonadjacent amino acids but juxtaposed by tertiary folding of protein. Epitopes formed from adjacent amino acids are typically retained after exposure to denaturing solvent, however epitopes formed via tertiary folding are typically lost after treatment with denaturing solvent. Epitopes usually have a unique spatial conformation, including at least 3-15 amino acids. Methods for determining which epitope is bound by a given antibody are well known in the art, including immunoblotting and immunoprecipitation assays and the like. Methods for determining the spatial conformation of an epitope include techniques in the art and techniques described herein, such as X-ray crystallography and two-dimensional nuclear magnetic resonance and the like.

As used herein, the terms "specifically bind" and "selectively bind" refer to the binding of an antibody to an epitope on a predetermined antigen. Typically, when recombinant human CD27 is used as an analyte and an antibody is used as a ligand, the antibody binds to a predetermined antigen at an equilibrium dissociation constant (K_{D}) of less than about 10⁻⁷ M or less measured by surface plasmon resonance (SPR) techniques in an instrument. Its affinity for binding to a predetermined antigen is at least twice when compared to its affinity for binding to a non-specific antigen other than the predetermined antigen or closely related antigen (such as BSA, etc). The term "antibody recognizing an antigen" can be used interchangeably herein with the term "specifically bound antibody".

The term "cross-reaction" refers to the ability of an antibody of the present invention to bind to CD27 from a different species. For example, an antibody of the present invention that binds to human CD27 can also bind to CD27 from another species. Cross-reactivity is measured by detecting specific reactivity with purified antigens in binding assays (e.g., SPR and ELISA), or binding or functional interactions with cells that express CD27 physiologically. Methods for determining cross-reactivity include standard binding assays as described herein, such as surface plasmon resonance (SPR) analysis, or flow cytometry.

The terms "inhibiting" or "blocking" are used interchangeably and encompass both partial and complete inhibition/blocking. Preferably, inhibition/blocking of CD70 reduces or alters the normal level or type of activity when CD70 binding occurs without inhibition or blocking. Inhibition and blocking are also intended to include any measurable decrease of CD70 binding affinity when contacted with an anti-CD27 antibody, compared to that when not contacted with an anti-CD27 antibody.

The term "inhibiting growth" (e.g., involving cells) is intended to include any measurable decrease in cell growth.

The terms "inducing an immune response" and "enhancing an immune response" are used interchangeably and refer to the stimulation (i.e., passive or adaptive) of an immune response to a particular antigen. When it comes to CDC or ADCC, the term "induction" refers to the stimulation of particular direct cytotoxic mechanism.

As used herein, the term "ADCC", namely antibody-dependent cell-mediated cytotoxicity, refers to that the cells expressing Fc receptors directly kill the target cells coated by an antibody by recognizing the Fc segment of the antibody. ADCC effector function of the antibody can be reduced or eliminated by modifying the Fc segment of IgG. The modification refers to mutations in antibody heavy chain constant region, such as mutations selected from N297A, L234A, L235A in IgG1; IgG2/4 chimera; or F235E or L234A/E235A mutations in IgG4.

Methods for producing and purifying antibodies and antigen-binding fragments are well known in the art and can be found, for example, in Antibody Experimental Technology Guide of Cold Spring Harbor, Chapters 5-8 and 15. For example, mice can be immunized with human CD27, or fragments thereof, and the resulting antibodies can then be re-natured, purified and sequenced by using conventional methods well known in the art. Antigen-binding fragments can also be prepared by conventional methods. The antibody or the antigen-binding fragment of the present invention is genetically engineered to introduce one or more human framework regions (FRs) to a non-human derived CDR. Human FR germline sequences can be obtained from ImMunoGeneTics (IMGT) via their website http://imgt.cines.fr, or from The Immunoglobulin FactsBook, 2001ISBN012441351.

The engineered antibody or antigen-binding fragment of the present invention may be prepared and purified using conventional methods. For example, cDNA sequence encoding corresponding anibody may be cloned and recombined into a GS expression vector. The recombinant immunoglobulin expression vector may then stably transfect CHO cells. As a more recommended method well known in the art, mammalian expression system will result in glycosylation of antibody, typically at the highly conserved N-terminal in the FC region. Stable clones are obtained through expression of an antibody specifically binding to human antigen. Positive clones may be expanded in a serum-free culture medium for antibody production in bioreactors. Culture medium, into which an antibody has been secreted, may be purified and collected by conventional techniques. The antibody may be filtered and concentrated using common techniques. Soluble mixture and aggregate may be effectively removed by common techniques, including size exclusion or ion exchange. The obtained product may be immediately frozen, for example at -70°C, or may be lyophilized.

The antibody of the present invention refers to a monoclonal antibody. The monoclonal antibody (mAb) of the present invention refers to an antibody obtained from a single clone of cell strain, and the cell strain is not limited to a eukaryotic, prokaryotic or phage clonal cell strain. Monoclonal antibodies or antigen-binding fragments can be obtained recombinantly using, for example, hybridoma techniques, recombinant technique, phage display technique, synthetic technique (e.g., CDR-grafting), or other techniques in the prior art.

"Administration" and "treatment," as it applies to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administration" and "treatment" can refer, e.g., to therapeutic, pharmacokinetic, diagnostic, research, and experimental methods. Treatment of a cell encompasses contacting a reagent with the cell, as well as contacting a reagent with a fluid, wherein the fluid is in contact with the cell. "Administration" and "treatment" also means *in vitro* and *ex vivo* treatments, e.g., of a cell, by a reagent, diagnostic, binding composition, or by another cell. "Treatment," as it applies to a human, veterinary, or a subject to be studied, refers to therapeutic treatment, prophylactic or preventative measures, research and diagnostic applications.

"Treat" means to administer a therapeutic agent, such as a composition comprising any of the binding compounds of the present invention, internally or externally to a patient having one or more disease symptoms for which the agent has known therapeutic activity. Typically, the agent is administered in an amount effective to alleviate one or more disease symptoms in the treated patient or population, regardless of by inducing the regression of such symptom(s) or by inhibiting the progression to any clinically measurable degree. The amount of a therapeutic agent that is effective to alleviate any particular disease symptom (also referred to "therapeutically effective amount") may vary according to factors such as the disease state, age, and weight of the patient, and the ability of the agent to elicit a desired response in the patient. Whether a disease symptom has been alleviated can be assessed by any clinical measurement typically used by physicians or other skilled healthcare providers to assess the severity or progression status of that symptom. Even if an embodiment of the present invention (e.g., a treatment method or article of manufacture) is not effective in alleviating the disease symptom(s) of interest in every patient, it does alleviate the target disease symptom(s) of interest in a statistically significant number of patients, as determined by any statistical test known in the art such as the Student's t-test, the chi-square test, the U-test according to Mann and Whitney, the Kruskal-Wallis test (H-test), Jonckheere-Terpstra-test and the Wilcoxon-test.

The term "consisting essentially of" or variations thereof, as used throughout the specification and claims, includes all such elements or groups of the elements, and optionally includes other elements that are in nature similar to or different from the elements, said other elements do not significantly alter the essential or novel properties of the specified dosage regimen, method or composition. As a non-limiting example, a binding compound consisting essentially of the recited amino acid sequence may also include one or more amino acids that do not significantly affect the properties of the binding compound.

The term "naturally occurring" when applied to a subject matter according to the present invention refers to the fact that the subject matter can be found in nature. For example, polypeptide sequences or polynucleotide sequences that are present in organisms (including viruses) that can be isolated from natural sources and that have not been intentionally modified artificially in laboratory, are naturally occurring.

"Effective amount" encompasses an amount sufficient to ameliorate or prevent a symptom or sign of a medical condition. Effective amount also means an amount sufficient to allow or facilitate diagnosis. An effective amount for a particular patient or veterinary subject may vary depending on factors such as the condition being treated, the general health of the patient, the route and dose of administration and the severity of side effects. An effective amount can be the maximal dose or dosing protocol that avoids significant side effects or toxic effects.

"Exogenous" refers to substances that are produced outside an organism, cell, or human body, depending on the background. "Endogenous" refers to substances that are produced within a cell, organism, or human body, depending on the background.

As used herein, the expressions "cell," "cell line," and "cell culture" are used interchangeably and all such designations include its progeny. Thus, the words "transformant" and "transformed cell" include the primary subject cell and cultures derived therefrom without considering the number of transfers. It is also understood that all progeny may not be precisely identical in the aspect of DNA content, due to deliberate or inadvertent mutations. Mutant progeny that have the same function or biological activity as that of originally transformed cell are also included. Where distinct designations are intended, it will be clear from the context.

"Optional" or "optionally" means that the event or situation that follows may but not necessarily occur, and the description will indicate the instances in which the event or circumstance occurs or does not occur. For example, "optionally comprises 1-3 antibody heavy chain variable regions" means the antibody heavy chain variable region with specific sequence can be, but not necessarily, present.

"Pharmaceutical composition" refers to a mixture comprising one or more compounds according to the present invention or a physiologically/pharmaceutically acceptable salt or prodrug thereof ,and other chemical components, such as physiologically/pharmaceutically acceptable carriers and excipients. The pharmaceutical composition aims at promoting the administration to an organism, facilitating the absorption of the active ingredient and thereby exerting a biological effect.

Hereinafter, the present invention is further described with reference to the examples. However, the scope of the present invention is not limited thereto. In the examples of the present invention, where specific conditions are not indicated, the experiments are generally conducted under conventional conditions as described in Antibody Technology Laboratory Manual and Molecular Cloning Manual of Cold Spring Harbor, or under the instruction proposed by the material or product manufacturers. Where the source of reagents is not specifically given, the reagents are commercially available conventional reagents.

### Example 1: Screening of human CD27 antigen-binding antibody fragment (Fab)

An *in vitro* phage display system was used for screening human derived antibody fragments against human CD27 antigen. The antigen used included CD27-HisFc (Sino Biological #10039-H03H), CD27-Fc (Sino Biological #10039-H31H) and CD27-His (Sino Biological #10039-H08B1). The antigen was directly absorbed and immobilized on a Maxisorp 96-well screening plate (Thermo Scientific 446469), or absorbed on Dynabeads microparticles (M280, Streptavidin, Invitrogen #60210) after being biotinylated, screening was performed by KingFisher (Thermo Scientific) automated screening workstation using standard phage display screening procedures. More than 3-4 rounds of screening were performed for each antigen, and the human antibody Fc fragment was used in each round to eliminate background. After the last round of screening, single colony was selected for cloning and incubation, the supernatant was collected, and antibody fragments that bind to human CD27 were primarily identified by ELISA. The primary selection criteria was that the ELISA readings were at least twice higher than the background signal.

### Example 2: Determination of the affinity of antibody fragment to human CD27 by Biofilm Interference (BLI)

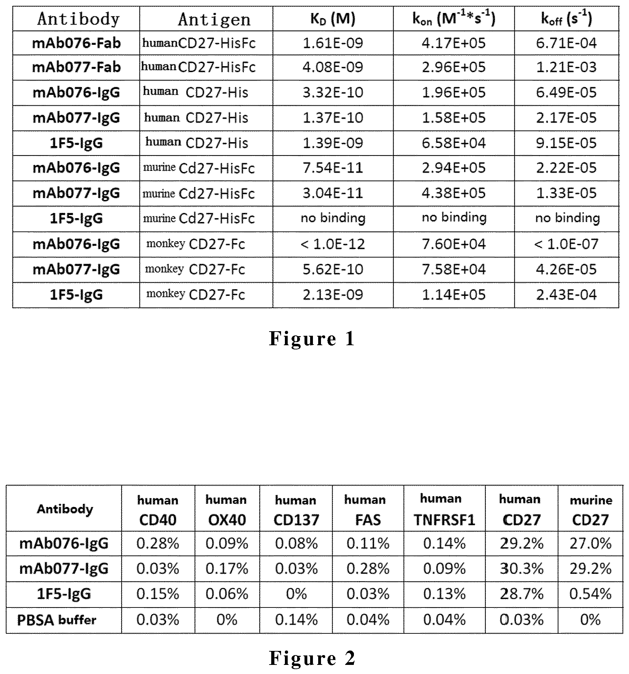

Histidine tag was added to each unique sequence of the antibody Fab fragment at the C-terminal, and the antibody Fab fragment was overexpressed in *E. coli* expression system, and then purified by using Ni-NTA affinity resin. The affinity of Fab to human CD27 was determined by the following method using the ForteBio Octet RED96 system: the human CD27-HisFc fusion protein was firstly captured using the AHC sensor of the BLI system (specific for human IgG antibody Fc fragment), then immersed in assay buffer containing 5-10 µg/ml of each purified Fab protein. The collected data was processed by Data Acquisition 7.1 software and fitted to a 1:1 combined Langmuir model curve. The antibodies mAb076 and mAb077 are the two antibodies with optimal binding and were selected for further analysis and development experiments. The kinetic constants for their binding to CD27 are shown in Figure 1.

The sequences for heavy and light chain variable region of human mAb mAb076 are as follows:
mAb076 VH 
EVQLVESGGGLVQPGGSLRLSCAASGFTFSDYGIHWVRQAPGKGLEWIGWINP
NRGSTNYAQKFQGRVTISRDNSKNTLYLQLNSLRAEDTAVYYCARDYGYTWYF
DVWGQGTLVTVSS SEQ ID NO: 1
mAb076 VL

DIQLTQSPSSLSASVGDRVTITCRASQDISSVLAWYQQKPGKAPKLLIYAASSLQS
GVPSRFSGSGSGTDFTLTISSLQPEDFATYYCQQRDAWPLTFGQGTKVEIK
SEQ ID NO: 2

They contain the following CDR sequences:

| Name | Sequence | NO. |
|---|---|---|
| HCDR1 | DYGIH | SEQID NO: 3 |
| HCDR2 | WINPNRGSTNYAQKFQG | SEQID NO: 4 |
| HCDR3 | DYGYTWYFDV | SEQID NO: 5 |
| LCDR1 | RASQDISSVLA | SEQID NO: 6 |
| LCDR2 | AASSLQS | SEQID NO: 7 |
| LCDR3 | QQRDAWPLT | SEQID NO: 8 |

The sequences for heavy and light chain variable region of human mAb mAb077 are as follows:
mAb077 VH 
EVQLVESGGGLVQPGGSLRLSCAASGFTFSGYGIHWVRQAPGKGLEWIGWINP
NRGSTKYAQKFQGRVTISRDNSKNTLYLQLNSLRAEDTAVYYCARDPGYTWYF
DVWGQGTLVTVSS SEQ ID NO：9
mAb077 VL

DIQLTQSPSSLSASVGDRVTITCRASQDISSDLAWYQQKPGKAPKLLIYAASTLQS
GVPSRFSGSGSGTDFTLTISSLQPEDFATYYCQQRDAWPPTFGQGTKVEIK
SEQ ID NO: 10

It contains the following CDR sequences:

| Name | Sequence | NO |
|---|---|---|
| HCDR1 | GYGIH | SEQID NO: 11 |
| HCDR2 | WINPNRGSTKYAQKFQG | SEQID NO: 12 |
| HCDR3 | DPGYTWYFDV | SEQID NO: 13 |
| LCDR1 | RASQDISSDLA | SEQID NO: 14 |
| LCDR2 | AASTLQS | SEQID NO: 15 |
| LCDR3 | QQRDAWPPT | SEQID NO: 16 |

### Example 3: Conversion of antibody Fab fragment to IgG and its expression and purification

Each antibody fragment was respectively cloned into a mammalian cell expression vector which expresses the light chain and heavy chain of the IgG1 subtype antibody. The paired vectors were transfected into HEK293 cells in a transient transfection manner according to standard procedures. The supernatant was collected after expression, and subjected to centrifugation and filtration, and then IgG was purified by protein A affinity chromatography. The eluted proteins were neutralized and ion exchanged into PB buffer (20 mM sodium phosphate, 150 mM NaCl, pH 7.0). The protein concentration is determined by an ultraviolet spectrophotometer and its purity and properties are determined under denaturing, reducing or non-reducing conditions.

### Example 4: Determining the binding of antibodies to CD27 from different species by surface plasmon resonance (SPR)

Affinity and kinetic parameters of antibody binding to human CD27 were determined using Biacore™ T200. The anti-human IgG (Fc) antibody was first immobilized on the CM5 chip of the Biacore system, and the IgG1 portion of the mAb076, mAb077 and the reference antibody 1F5 was captured (flow rate 10 µL/min, 12 seconds). In subsequent kinetic experiments, different concentrations of human CD27 antigen (3.13, 6.25, 12.5, 25, 50, 100 nM) were analyzed at 30 µL/min for 300 seconds, and then dissociation for 300 seconds. The system buffer was 1×HBS-EP (10 mM HEPES, 150 mM NaCl, 3 mM EDTA, 0.005% v/v P20 surfactant, pH 7.4, 25 °C, the same in experiments below), a chip in which no protein was immobilized was set as a blank control for each experiment. At the end of each experiment, the chip was regenerated with 10 mM glycine (pH 1.5) at a flow rate of 30 µL/min for 30 seconds. Experimental data was processed by BIAevaluation 3.2 software and fitted to a 1:1 combined Langmuir model curve. The results are shown in Figure 1.

The affinity of the antibody binding to human CD27 was also determined using SPR. In this experiment, the murine CD27-FcHis fusion protein was directly immobilized on a CM5 chip (600 RU) by amino coupling. Antibodies at different concentrations (0.39, 0.78, 1.56, 3.13, 6.25, 12.5, 25, 50, 100 nM) were loaded at a flow rate of 30 µL/min for 30 seconds with a dissociation time of 1800 seconds. The results showed that both mAb076 and 077 had pM-level affinity for mouse CD27, whereas the reference antibody 1F5 did not. The data is shown in Figure 1.

Affinity and kinetic parameters of antibody binding to monkey CD27 were determined using a ForteBio Octet RED96. After biotinylation of each IgG antibody, the antibodies were immobilized on a Streptavidin-containing biosensor by means of EZ-Link Sulfo-NHS-Biotinylation Kit (Thermo Fisher Scientific). The sensor was then immersed in a buffer containing rhesus monkey CD27-Fc protein for binding for 300 seconds, and later dissociated in the blank buffer for 300 seconds. The collected data was processed by Data Acquisition 7.1 software and fitted to a 1:1 combined Langmuir model curve. As shown in Figure 1, the affinity of mAb076 and mAb077 is higher than that of 1F5.

### Example 5: Determination of specific binding of antibodies to human/murine CD27 expressed on cells

The binding of antibody to human or murine CD27 expressed on cell surface is determined by fluorescence activated cell sorting (FACS). The corresponding antigen was transiently transfected into HEK293 cells using a vector plasmid and expressed. After 48 hours, cells were harvested and washed once with cold FACS buffer (PBS, 1% BSA), and then incubated with 100 nM corresponding antibody for 1 hour on ice. After washing twice with buffer, the cells were bound to Alexa Fluor 647-conjugated murine anti-human Fc antibody for 30 minutes on ice. After washing once, the cells were loaded onto a Beckman® CytoFlex flow cytometer for detection and the percentage of cells with binding signals was calculated. As shown in Figure 2, the antibodies mAb076 and mAb077 bind significantly to human or murine CD27 on the cell surface, but have no binding to the other TNF receptor superfamily member antigen proteins including human or murine CD40, OX40, CD137, FAS, TNFRSF1A.

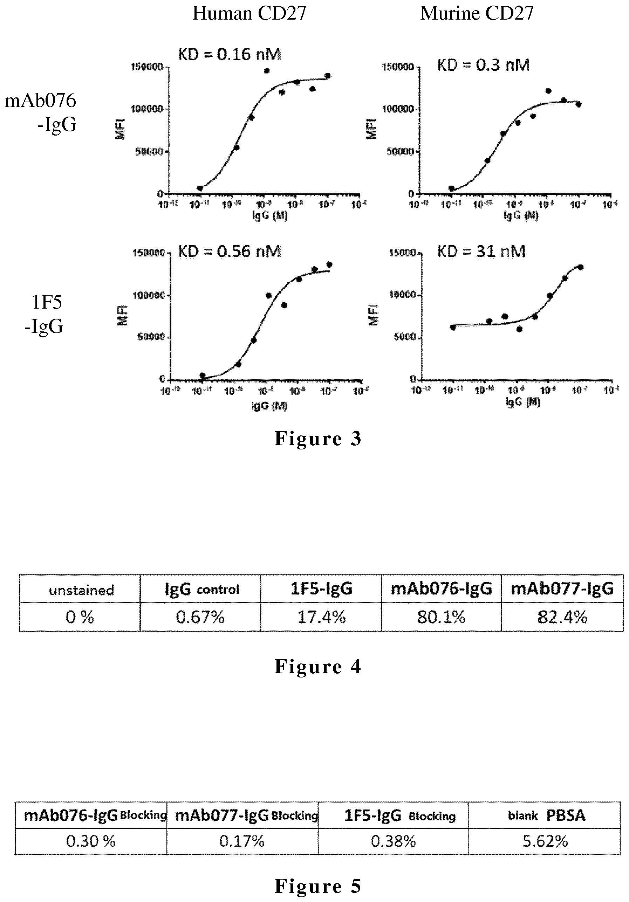

The EC50 of mAb076 antibody binding to human or murine CD27 expressed on the cell surface was determined by a 1:3 stepwise gradient dilution curve (100 nM to 0.05 nM). As shown in Figure 3, mAb076 and reference antibody 1F5 strongly bind to human CD27 expressed on cell surface, while 1F5 weakly binds to murine CD27.

### Example 6: Determination of binding of antibodies to mouse T cells

This antibody binding assay was performed using activated mouse spleen T cells. BALB/c spleen T cells were purified using mouse T cell isolation kit from Stemcell Technologies and activated with 50 ng/ml PMA and 1 µg/ml ionomycin for 2 days, then 10⁵ cells were collected from each sample and incubated on ice for 1 hour with 50 µl of 100 nM corresponding antibody. The cells were washed once with FACS buffer and stained with 100 µl of 1:500 diluted mouse anti-human Fc-APC (Biolegend) for 30 minutes on ice. The cells were washed once more with buffer and resuspended, then loaded onto a CytoFlex flow cytometer for detection and the percentage of cells with binding signals was calculated. As shown in Figure 4, both mAb076 and mAb077 strongly bind to activated mouse T cells.

### Example 7: CD27 ligand blocking assay of antibodies

The test that antibodies block the binding of CD27 to its ligand CD70 was detected by immunofluorescence staining and FACS. Transient transfection of CD27 into HEK293 cells using vector plasmid was performed. After 48 hours, the cells were harvested and washed once with cold FACS buffer, and then incubated with 100 nM of the corresponding antibody on ice for 1 hour, and 16 nM biotinylated labeled CD70 was added. After 30 minutes of incubation, the cells were washed twice with buffer and incubated with Alexa Fluor 647-conjugated streptavidin for 30 minutes. The cells were washed once more with buffer and resuspended, and then loaded onto a CytoFlex flow cytometer for detection, and the percentage of cells having CD70-specific binding signal was calculated. As shown in Figure 5, mAb076, mAb077 and the reference antibody are effective in blocking the binding of CD27 to CD70.

### Example 8: Activation of NF-kB cell signaling pathway by anti-CD27 antibody

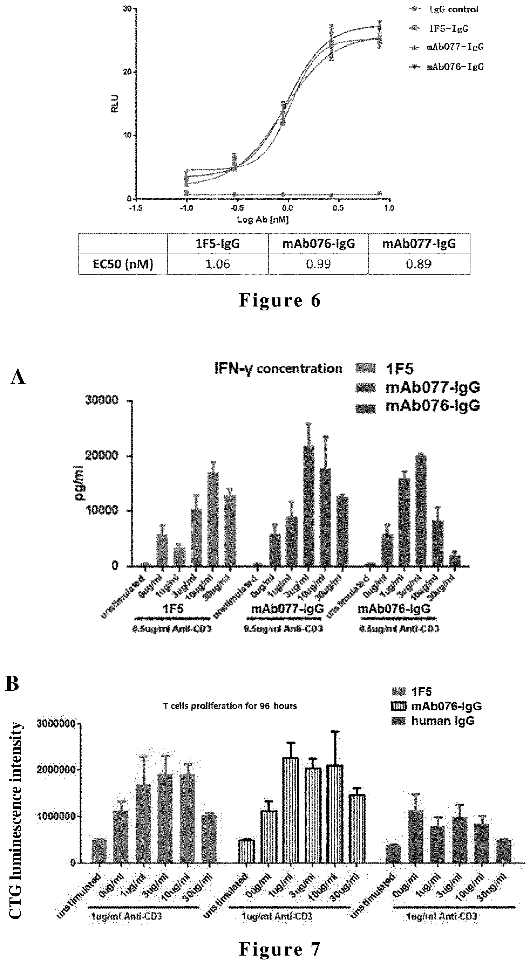

Activation of the cell surface CD27 molecule triggers activation of a series of downstream signaling pathway, including NK-kB, which can be detected by the luciferase reporter gene method. In this example, a plasmid expressing the human CD27 gene, the NFkB-luc and renilla luciferase reporter gene (Promega) were transfected into 5x10⁵ HEK293T cells. After 4 hours, HEK293T cells were loaded into 96-well plates at 2x10⁴ cells per well. After pre-mixing the gradient diluted corresponding antibody with F(ab')2 goat anti-human IgG Fcγ secondary antibody, the mixture was added into HEK293T cells and incubated for 18 hours in a 37 °C incubator with 5% CO₂. The cells were lysed and luciferase intensities were measured on SpectraMax i3x plate reader using Promega's Dual-luciferase kit. Relative luciferase units (RLU) were analyzed and mapped using GraphPad Prism software. As shown in Figure 6, both mAb076 and mAb077 have significant effect on activation of the NF-kB reporter gene.

### Example 9: T cell activation assay of anti-CD27 antibody

In the T cell activation function experiment, 96-well cell culture plates were pre-treated with 100 ul of 0.5 ug/ml anti-CD3 antibody and various concentrations of the test antibodies (30, 10, 3, 1, 0 ug/ml) overnight at 4 °C. Human peripheral blood mononuclear cells (PBMC) were isolated using Histopaque 1077 kit from Sigma, then purified using Stemcell Technologies' purification kit and resuspended in RPMI 1640 medium containing 10% FBS at 2.5 x 10⁵/ml. 200 ul (5 x 10⁴) of the above cells were added into each well of the 96-well plate, and 5 ug/ml of anti-CD28 stimulating antibody was used in parallel as a positive control. After incubating the cells for 4 days in a 37 °C incubator with 5% CO₂, 100 µl of each well was transferred to a new sample plate (the remaining cells were used for IFN-γ detection). 80 ul of CellTiter-Glo® reagent was added and incubated for 10 minutes to stabilize the signal, then the intensity of luminescence was detected using SpectraMax i3x plate reader. As shown in Figure 7, mAb076 and mAb077 in the form of IgG1 promoted the expression of T cell IFN-y more than the reference antibody 1F5. In another experiment, the mAb076 antibody also activated T cells more than corresponding 1F5 form.

### Example 10: Determining the binding of antibody to FcγR receptor by SPR

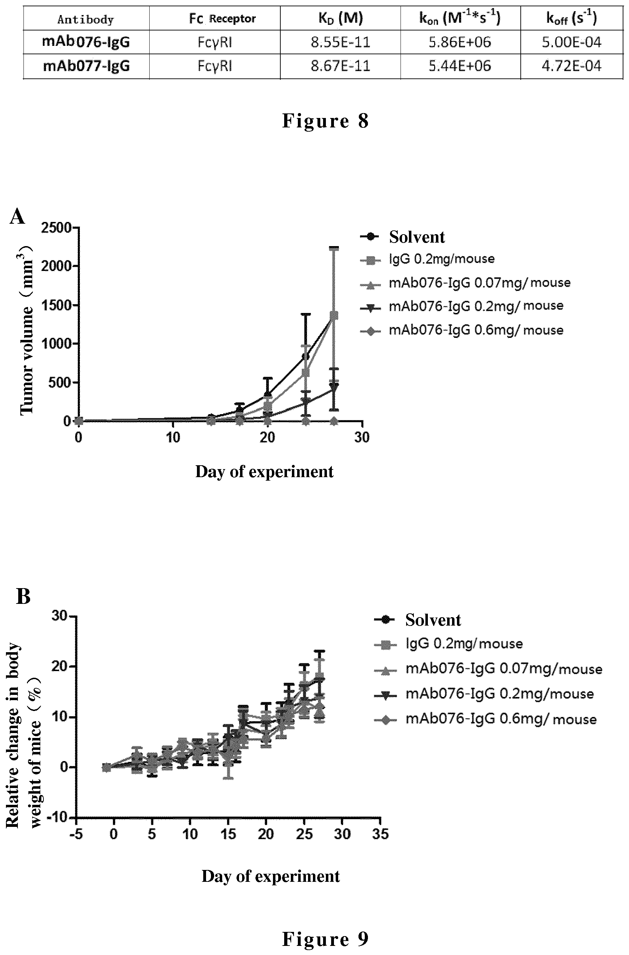

Binding of antibodies to FcγR receptor was detected by SPR method on Biacore™ T200. Protein L was immobilized on a CM5 sensor chip for capture of antibodies (10 µl/min, loading for 12 sec). Then, different concentrations of FcγRI receptor (0.39, 0.78, 1.56, 3.13, 6.25nM in system buffer) were loaded for 300 seconds at 10µl/min and dissociated for 600 seconds. For FcγRIIa (CD32a) or FcγRIIb (CD32b) receptors, the same method was used, but at concentrations of 25, 50, 100, 200, 400 nM. The collected data was processed by Data Acquisition 7.1 software and fitted to a 1:1 combined Langmuir model curve. mAb076 and mAb077 bind only to the FcγRI receptor, but not to FcγRIIa and FcγRIIb. The data of results are shown in Figure 8.

### Example 11: Efficacy of mAb076-IgG in a murine tumor model

40 C57BL/6 mice, female, 5-6 week-old, 18-20g, were divided into 5 groups, 8 mice in each group. E.G7-OVA cells were cultured *in vitro,* and subcutaneously transplanted into C57BL/6 mice under sterile conditions, and each mouse was inoculated with 1×10⁴ cells. The day of tumor inoculation was set as day 0. The first administration was started on the third day after inoculation, and then proceeded as follows:
G1: solvent control group;
G2: IgG1 0.2mg/mouse, i.p.,N=8, Q2D, Day 3, 5, 7, 9, 11, 13;
G3: mAb076-IgG 0.07mg/mouse, i.p.,N=8, Q2D, Day 3, 5, 7, 9, 11, 13;
G4: mAb076-IgG 0.2mg/mouse, i.p.,N=8, Q2D, Day 3, 5, 7, 9, 11, 13;
G5: mAb076-IgG 0.6mg/mouse, i.p.,N=8, Q2D, Day 3, 5, 7, 9, 11, 13;

After administration the behavior of the animals was monitored daily for a total of 27 days. After measuring the long diameter and short diameter of the tumor with a vernier caliper, the tumor volume was calculated by length × width²/2. The tumor inhibition rate was calculated by the formula TGI% = (1-T/C) × 100%. T and C represent the tumor volume (TV) of the administration group and the control group at a specific time point, respectively. The change in body weight was calculated using the formula RCBW% = (BWᵢ - BW₀) / BW₀ × 100%, BWᵢ is the current body weight of the mouse, and BW₀ is the body weight of the mouse on the day of grouping. All experimental results are expressed as mean tumor volume ± standard error (SEM). The T-Test method was used to compare whether the tumor volume and tumor weight of the treatment group were significantly different from those of the control group. All data were analyzed using Graphpad, p < 0.05 for significant differences. As shown in Figure 9, in the E.G7-OVA tumor model, mAb076-IgG showed anti-tumor effect, and at the end of experiment the average tumor volumes of each group were: 1355.3 mm³, 1365.87 mm³, 0 mm³, 404.88 mm³, 0 mm³, respectively. The tumor growth inhibition (TGI) of the low, medium and high dose groups was 100%, 70.36% and 100%, respectively. There was no significant decrease in body weight of C57BL/6 mice in each of the administration groups, indicating that C57BL/6 mice were well tolerated to mAb076-IgG at these doses.

### Example 12: Efficacy of mAb077-IgG in a xenograft tumor model

45 CB17-SCID mice, female, 5-6 week-old, 18-20g, purchased from Beijing Vital River, were divided into 5 groups, 9 mice in each group. Raji cells were cultured *in vitro* in RPMI-1640 medium containing 10% fetal bovine serum (FBS). Raji cells in exponential growth phase were collected and subcutaneously transplanted into CB17-SCID mice under sterile conditions, and each mouse was inoculated with 2 x 10⁶ cells. The day of tumor inoculation was set as day 0. The first administration was started on the third day after inoculation, and then proceeded as follows:
Group 1: PBS solvent, i.p.,N=9, Q2D, Day 3, 5, 7, 9, 11, 13, 15;
Group 2: IgG1 30mg/kg, i.p.,N=9, Q2D, Day 3, 5, 7, 9, 11, 13, 15;
Group 3: mAb077-IgG 3mg/kg, i.p.,N=9, Q2D, Day 3, 5, 7, 9, 11, 13, 15;
Group 4: mAb077-IgG 10mg/kg, i.p.,N=9, Q2D, Day 3, 5, 7, 9, 11, 13, 15;
Group 5: mAb077-IgG 30mg/kg, i.p.,N=9, Q2D, Day 3, 5, 7, 9, 11, 13, 15;

After administration the behavior of the animals was monitored daily for a total of 18 days. After measuring the long diameter and short diameter of the tumor with a vernier caliper, the tumor volume was calculated by length × width²/2. The tumor inhibition rate was calculated by the formula TGI% = (1-T/C) × 100%. T and C represent the tumor volume (TV) of the administration group and the control group at a specific time point, respectively. The change in body weight was calculated using the formula RCBW% = (BWᵢ - BW₀) / BW₀ × 100%, BWᵢ is the current body weight of the mouse, and BW₀ is the body weight of the mouse on the day of grouping. All experimental results were expressed as mean tumor volume ± standard error (SEM). The T-Test method was used to compare whether the tumor volume and tumor weight of the treatment group were significantly different from those of the control group. All data was analyzed using Graphpad, p < 0.05 for significant differences.

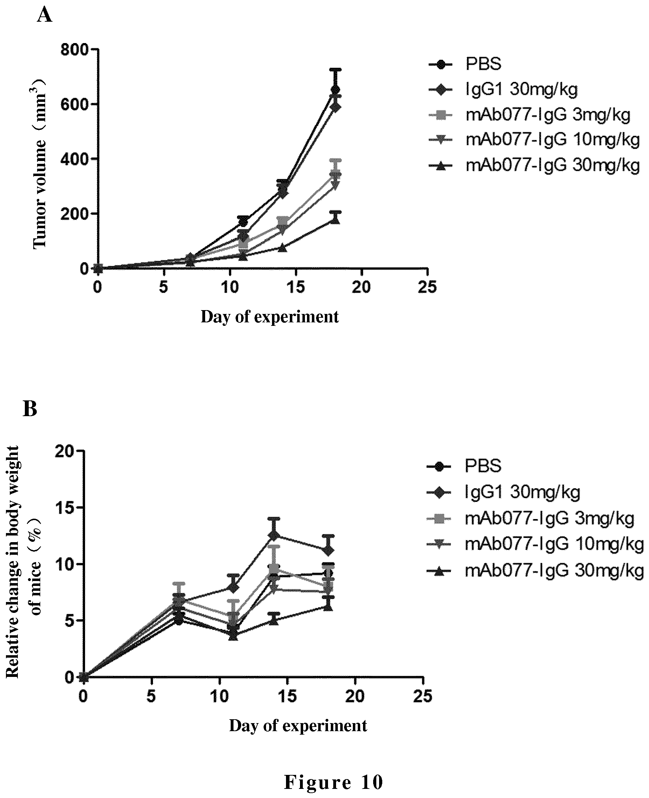

As shown in Figure 10, mAb077-IgG shows significant anti-tumor effect and dose-dependent effect in the Raji transplanted tumor model. The tumor growth inhibition (TGI) in the low, medium and high dose groups were 41%, 49% and 70%, respectively. There was no significant decrease in body weight of mice in each of the administration groups, indicating that mice were well tolerated to mAb077-IgG at these doses.

## Claims

1. An anti-CD27 antibody or antigen-binding fragment thereof comprising:
an antibody light chain variable region comprising at least one LCDR as shown in the sequences selected from SEQ ID NO: 6, SEQ ID NO: 7, SEQ ID NO: 8; SEQ ID NO : 14, SEQ ID NO: 15 or SEQ ID NO: 16; and
an antibody heavy chain variable region comprising at least one HCDR as shown in the sequences selected from SEQ ID NO: 3, SEQ ID NO: 4, SEQ ID NO: 5, SEQ ID NO :11, SEQ ID NO: 12 or SEQ ID NO: 13.

2. The anti-CD27 antibody or antigen-binding fragment thereof according to claim 1, wherein the antibody light chain variable region comprises LCDR1 as shown in SEQ ID NO: 6 or SEQ ID NO: 14.

3. The anti-CD27 antibody or antigen-binding fragment thereof according to claim 1, wherein the antibody light chain variable region comprises LCDR2 as shown in SEQ ID NO: 7 or SEQ ID NO: 15.

4. The anti-CD27 antibody or antigen-binding fragment thereof according to claim 1, wherein the antibody light chain variable region comprises LCDR3 as shown in SEQ ID NO: 8 or SEQ ID NO: 16.

5. The anti-CD27 antibody or antigen-binding fragment thereof according to claim 1, wherein the antibody heavy chain variable region comprises HCDR1 as shown in SEQ ID NO:3 or SEQ ID NO:11.

6. The anti-CD27 antibody or antigen-binding fragment thereof according to claim 1, wherein the antibody heavy chain variable region comprises HCDR2 as shown in SEQ ID NO:4 or SEQ ID NO:12.

7. The anti-CD27 antibody or antigen-binding fragment thereof according to claim 1, wherein the antibody heavy chain variable region comprises HCDR3 as shown in SEQ ID NO:5 or SEQ ID NO:13.

8. The anti-CD27 antibody or antigen-binding fragment thereof according to claim 1, wherein the antibody light chain variable region comprises LCDR1, LCDR2 and LCDR3 as shown in SEQ ID NO: 6, SEQ ID NO: 7 and SEQ ID NO: 8 respectively.

9. The anti-CD27 antibody or antigen-binding fragment thereof according to claim 1, wherein the antibody light chain variable region comprises LCDR1, LCDR2 and LCDR3 as shown in SEQ ID NO: 14, SEQ ID NO: 15 and SEQ ID NO: 16 respectively.

10. The anti-CD27 antibody or antigen-binding fragment thereof according to claim 1, wherein the antibody heavy chain variable region comprises HCDR1, HCDR2 and HCDR3 as shown in SEQ ID NO: 3, SEQ ID NO: 4 and SEQ ID NO: 5 respectively.

11. The anti-CD27 antibody or antigen-binding fragment thereof according to claim 1, wherein the antibody heavy chain variable region comprises HCDR1, HCDR2 and HCDR3 as shown in SEQ ID NO: 11, SEQ ID NO: 12 and SEQ ID NO: 13 respectively.

12. The anti-CD27 antibody or antigen-binding fragment thereof according to claim 1, wherein the antibody light chain variable region comprises:
LCDR1, LCDR2 and LCDR3 as shown in SEQ ID NO: 6, SEQ ID NO: 7 and SEQ ID NO: 8 respectively; or
LCDR1, LCDR2 and LCDR3 as shown in SEQ ID NO: 14, SEQ ID NO: 15 and SEQ ID NO: 16 respectively;
and wherein the antibody heavy chain variable region comprises:
HCDR1, HCDR2 and HCDR3 as shown in SEQ ID NO: 3, SEQ ID NO: 4 and SEQ ID NO: 5 respectively; or
LCDR1, LCDR2 and LCDR3 as shown in SEQ ID NO: 11, SEQ ID NO: 12 and SEQ ID NO: 13 respectively.

13. The anti-CD27 antibody or antigen-binding fragment thereof according to claim 12, wherein the antibody light chain variable region comprises LCDR1, LCDR2 and LCDR3 as shown in SEQ ID NO: 6, SEQ ID NO: 7 and SEQ ID NO: 8 respectively; the antibody heavy chain variable region comprises HCDR1, HCDR2 and HCDR3 as shown in SEQ ID NO: 3, SEQ ID NO: 4 and SEQ ID NO: 5 respectively.

14. The anti-CD27 antibody or antigen-binding fragment thereof according to claim 12, wherein the antibody light chain variable region comprises LCDR1, LCDR2 and LCDR3 as shown in SEQ ID NO: 14, SEQ ID NO: 15 and SEQ ID NO: 16 respectively; the antibody heavy chain variable region comprises LCDR1, LCDR2 and LCDR3 as shown in SEQ ID NO: 11, SEQ ID NO: 12 and SEQ ID NO: 13 respectively.

15. The anti-CD27 antibody or antigen-binding fragment thereof according to claim 12, wherein the sequence of the antibody light chain variable region is selected from SEQ ID NO: 2 or SEQ ID NO: 10; the sequence of heavy chain variable region is selected from SEQ ID NO: 1 or SEQ ID NO: 9.

16. The anti-CD27 antibody or antigen-binding fragment thereof according to claim 12, wherein the sequence of the antibody light chain variable region is SEQ ID NO: 2, the sequence of the heavy chain variable region is SEQ ID NO: 1.

17. The anti-CD27 antibody or antigen-binding fragment thereof according to claim 12, wherein the sequence of the antibody light chain variable region is SEQ ID NO: 10, the sequence of the heavy chain variable region is SEQ ID NO: 9.

18. The anti-CD27 antibody or antigen-binding fragment thereof according to any one of claims 1 to 17, wherein the antibody or antigen-binding fragment thereof is a murine antibody or fragment thereof.

19. The anti-CD27 antibody or antigen-binding fragment thereof according to any one of claims 1 to 17, wherein the antibody or antigen-binding fragment thereof is a chimeric antibody or fragment thereof.

20. The anti-CD27 antibody or antigen-binding fragment thereof according to any one of claims 1 to 17, wherein the antibody or antigen-binding fragment thereof is a humanized antibody or fragment thereof.

21. The anti-CD27 antibody or antigen-binding fragment thereof according to any one of claims 1 to 17, wherein the antibody or antigen-binding fragment thereof is a human antibody or fragment thereof.

22. The anti-CD27 antibody or antigen-binding fragment thereof according to claim 21, wherein the sequence of the antibody light chain variable region sequence is SEQ ID NO: 2 or SEQ ID NO: 10 or a variant thereof; the variant preferably has 0-10 amino acid change(s) in the light chain variable region.

23. The anti-CD27 antibody or antigen-binding fragment thereof according to claim 21, wherein the sequence of the antibody heavy chain variable region is SEQ ID NO: 1 or SEQ ID NO: 9 or a variant thereof; the variant preferably has 0-10 amino acid change(s) in the heavy chain variable region.

24. A DNA sequence encoding the antibody of any one of claims 1-23.

25. An expression vector comprising the DNA sequence of claim 24.

26. A host cell transformed with the expression vector of claim 25.

27. The host cell according to claim 25, **characterized in that** the host cell is bacterium, preferably is *E. coli.*

28. The host cell according to claim 25, **characterized in that** the host cell is yeast, preferably is *pichia pastoris.*

29. The host cell according to claim 25, **characterized in that** the host cell is mammalian cell, preferably is Chinese hamster ovary (CHO) cell or human embryonic kidney (HEK) 293 cell.

30. A pharmaceutical composition comprising the anti-CD27 antibody or antigen-binding fragment thereof of any one of claims 1-17 and a pharmaceutically acceptable excipient, dilution or carrier.

31. A multispecific antibody comprising the light chain variable region and the heavy chain variable region as defined in any one of claims 1-17.

32. A single chain antibody comprising the light chain variable region and the heavy chain variable region as defined in any one of claims 1-17.

33. An antibody-drug conjugate, wherein the antibody comprises the light chain variable region and the heavy chain variable region as defined in any one of claims 1-17.

34. Use of the anti-CD27 antibody or antigen-binding fragment thereof according to any one of claims 1 to 17, or the pharmaceutical composition according to claim 30, or the multispecific antibody according to claim 31, or the single chain antibody according to claim 32, or the antibody-drug conjugate according to claim 33 in the preparation of a medicament for treating or preventing a CD27-mediated disease or condition; wherein, the disease is preferably cancer and autoimmune disease; more preferably is CD27-expressing cancer and autoimmune disease; most preferably, the cancer is rectal cancer, brain cancer, kidney cancer, lung cancer, liver cancer, multiple myeloma and melanin; most preferably, the autoimmune disease is autoimmune encephalomyelitis, systemic lupus erythematosus, multiple sclerosis and rheumatoid arthritis.

35. A method of treating and preventing CD27-mediated disease or condition, the method comprising administering therapeutically effective amount of the anti-CD27 antibody or antigen-binding fragment thereof according to any one of claims 1 to 17, or the pharmaceutical composition according to claim 30, or the multispecific antibody according to claim 31, or the single chain antibody according to claim 32, or the antibody-drug conjugate according to claim 33 to the patients in need thereof; wherein, the disease is preferably cancer and autoimmune disease; more preferably is CD27-expressing cancer and autoimmune disease; most preferably, the cancer is rectal cancer, brain cancer, kidney cancer, lung cancer, liver cancer, multiple myeloma and melanin; most preferably, the autoimmune disease is autoimmune encephalomyelitis, systemic lupus erythematosus, multiple sclerosis and rheumatoid arthritis.


Sequence Listing

<110>  JIANGSU HENGRUI MEDICINE CO., LTD.

<120>  ANTI-CD27 ANTIBODY, ANTIGEN-BINDING FRAGMENT THEREOF, AND MEDICAL USE
OF SAME

<130>  360245CG

<160>  16

<170>  PatentIn version 3.5

<210>  1
<211>  119
<212>  PRT
<213>  Homo sapiens

<400>  1

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Ile His Trp Val Arg Gln Ala Pro Gly Lys Gly Leu Glu Trp Ile
        35                  40                  45

Gly Trp Ile Asn Pro Asn Arg Gly Ser Thr Asn Tyr Ala Gln Lys Phe
    50                  55                  60

Gln Gly Arg Val Thr Ile Ser Arg Asp Asn Ser Lys Asn Thr Leu Tyr
65                  70                  75                  80

Leu Gln Leu Asn Ser Leu Arg Ala Glu Asp Thr Ala Val Tyr Tyr Cys
                85                  90                  95

Ala Arg Asp Tyr Gly Tyr Thr Trp Tyr Phe Asp Val Trp Gly Gln Gly
            100                 105                 110

Thr Leu Val Thr Val Ser Ser
        115

<210>  2
<211>  107
<212>  PRT
<213>  Homo sapiens

<400>  2

Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Asp Ile Ser Ser Val
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Arg Asp Ala Trp Pro Leu
                85                  90                  95

Thr Phe Gly Gln Gly Thr Lys Val Glu Ile Lys
            100                 105

<210>  3
<211>  5
<212>  PRT
<213>  Homo sapiens

<400>  3

Asp Tyr Gly Ile His
1               5

<210>  4
<211>  17
<212>  PRT
<213>  Homo sapiens

<400>  4

Trp Ile Asn Pro Asn Arg Gly Ser Thr Asn Tyr Ala Gln Lys Phe Gln
1               5                   10                  15

Gly

<210>  5
<211>  10
<212>  PRT
<213>  Homo sapiens

<400>  5

Asp Tyr Gly Tyr Thr Trp Tyr Phe Asp Val
1               5                   10

<210>  6
<211>  11

<212>  PRT
<213>  Homo sapiens

<400>  6

Arg Ala Ser Gln Asp Ile Ser Ser Val Leu Ala
1               5                   10


<210>  7
<211>  7
<212>  PRT
<213>  Homo sapiens

<400>  7

Ala Ala Ser Ser Leu Gln Ser
1               5


<210>  8
<211>  9
<212>  PRT
<213>  Homo sapiens

<400>  8

Gln Gln Arg Asp Ala Trp Pro Leu Thr
1               5


<210>  9
<211>  119
<212>  PRT
<213>  Homo sapiens

<400>  9

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Gly Tyr
            20                  25                  30


Gly Ile His Trp Val Arg Gln Ala Pro Gly Lys Gly Leu Glu Trp Ile
        35                  40                  45


Gly Trp Ile Asn Pro Asn Arg Gly Ser Thr Lys Tyr Ala Gln Lys Phe
    50                  55                  60


Gln Gly Arg Val Thr Ile Ser Arg Asp Asn Ser Lys Asn Thr Leu Tyr
65                  70                  75                  80


Leu Gln Leu Asn Ser Leu Arg Ala Glu Asp Thr Ala Val Tyr Tyr Cys
                85                  90                  95


Ala Arg Asp Pro Gly Tyr Thr Trp Tyr Phe Asp Val Trp Gly Gln Gly

100                 105                 110

Thr Leu Val Thr Val Ser Ser
        115

<210>  10
<211>  107
<212>  PRT
<213>  Homo sapiens

<400>  10

Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Asp Ile Ser Ser Asp
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Arg Asp Ala Trp Pro Pro
                85                  90                  95

Thr Phe Gly Gln Gly Thr Lys Val Glu Ile Lys
            100                 105

<210>  11
<211>  5
<212>  PRT
<213>  Homo sapiens

<400>  11

Gly Tyr Gly Ile His
1               5

<210>  12
<211>  17
<212>  PRT
<213>  Homo sapiens

<400>  12

Trp Ile Asn Pro Asn Arg Gly Ser Thr Lys Tyr Ala Gln Lys Phe Gln
1               5                   10                  15

Gly

<210> 13
<211> 10
<212> PRT
<213> Homo sapiens

<400> 13

Asp Pro Gly Tyr Thr Trp Tyr Phe Asp Val
1               5                   10

<210> 14
<211> 11
<212> PRT
<213> Homo sapiens

<400> 14

Arg Ala Ser Gln Asp Ile Ser Ser Asp Leu Ala
1               5                   10

<210> 15
<211> 7
<212> PRT
<213> Homo sapiens

<400> 15

Ala Ala Ser Thr Leu Gln Ser
1               5

<210> 16
<211> 9
<212> PRT
<213> Homo sapiens

<400> 16

Gln Gln Arg Asp Ala Trp Pro Pro Thr
1               5